# NEUE EUROPÄISCHE PATENTSCHRIFT

(11) **EP 1 124 589 B2**
(45) Veröffentlichungstag und Bekanntmachung des Hinweises auf die Entscheidung über den Einspruch: **25.02.2009**
(45) Hinweis auf die Patenterteilung: 08.01.2003
(21) Anmeldenummer: 99950784.1
(22) Anmeldetag: 27.10.1999
(51) Int. Cl.: A61L 24/10

(54) **FIBRIN-GEWEBEKLEBER-FORMULIERUNG UND VERFAHREN ZU DESSEN HERSTELLUNG**
FIBRIN TISSUE ADHESIVE FORMULATION AND METHOD FOR THE PRODUCTION THEREOF
FORMULATION D'ADHESIF TISSULAIRE A BASE DE FIBRINE ET PROCEDE DE PRODUCTION ASSOCIE

(30) Priorität: 27.10.1998 DE 19849589
(43) Veröffentlichungstag der Anmeldung: 22.08.2001
(73) Patentinhaber: Glatt Process Technology GmbH, 79589 Binzen (DE)
(72) Erfinder: PRASCH, Armin, D-79100 Freiburg (DE); LUY, Bernhard, D-79102 Freiburg (DE)
(74) Vertreter: Pfenning, Meinig & Partner GbR
(86) Internationale Anmeldenummer: PCT/EP1999/008128
(87) Internationale Veröffentlichungsnummer: WO 2000/024436

(56) Entgegenhaltungen:
- WO-A-96/29990
- WO-A-97/44015
- WO-A-99/15637
- WO-A1-00/38752
- D.PRUNKARD: "Heterologous Production of recombinant human fibrinogen, thrombin, and favctor XIII as components of completely recombinant fibrin sealants" THROMBOSIS & HAEMOSTASIS, Bd. supp, Nr. 9, 6. September 1997 (1997-09-06), Seite 372 XP002112932

## Beschreibung

Die Erfindung betrifft eine geeignete Formulierung einer stabilen, pulverförmigen, möglichst staubfreien und dadurch gut rieselfähigen, festen Darreichungsform eines Fibrin-Gewebeklebers zum Einsatz bei der Blutstillung, Wundversorgung (-heilung), Gewebeklebung und Nahtsicherung bei äußeren und inneren chirurgischen Operationen am Menschen, wobei sich die Formulierung mittels eines Wirbelschichttrocknungsverfahrens darstellen läßt.

Die Blutgerinnung läuft im gesunden Körper bei Tieren (Säugetiere) und beim Menschen natürlich in Form einer Co-Enzym/Enzym gesteuerten Kaskadenreaktion ab. Der Hauptvorgang besteht darin, daß das (in Wasser, physiologischer Kochsalzlösung und auch im Blut) lösliche Fibrinogen in das unlösliche Fibrin übergeführt wird. Hierfür ist das proteolytische Enzym Thrombin notwendig, das durch den Prothrombinaktivator, einem Gemisch aus dem Stuart-Prower-Faktor (Faktor X) und dem Proaccelerin (Faktor V) bei Anwesenheit von Calcium-Ionen aus dem inaktiven Prothrombin (Faktor II) gebildet wird. Das Thrombin spaltet das in der Regel als Monomer (zu 75 %) mit einer Molmasse von 340.000 Dalton, als Di-Mer (zu 15 %) und als Polymer (zu 10 %) vorliegende Fibrinogen in Fibrin und bildet dadurch lange Molekülketten. Diese werden durch den fibrinstabilisierenden Faktor XIII (und bei Anwesenheit von Calcium-Ionen) zu einem stabilen, quervernetzten Fibrinpolymer verknüpft. Für diese biochemische Reaktion ist das reibungslose Zusammenspiel einer Reihe von Faktoren (Gerinnungsfaktoren) notwendig. Im gesunden Organismus liegen die benötigten Gerinnungsfaktoren in ausreichender Menge in einem labilen Gleichgewicht vor.

Störungen dieses Gleichgewichts können lebensgefährlich sein. Störungen des Gleichgewichts können neben dem erblichen Mangel von bereits einem Gerinnungsfaktor (z.B. Hämophilie), bei starken Gewebeblutungen, bei großflächigen, diffusen Blutungen (Weichgewebeblutungen), die nicht durch einen mechanischen Verschluß größerer arterieller oder venöser Gefäße gehemmt werden können, oder durch antikoagulativ wirkende, therapeutisch verabreichte Medikamente zur Thrombembolieprophylaxe verursacht werden. Diese Störungen können durch sog. Fibrin-Gewebekleber, einem Gemisch aus Fibrinogen, Faktor XIII, Thrombin und Human-Albumin sowie Calciumchlorid, ausgeglichen werden, wodurch es zu einer lokalen Blutstillung kommt. Fibrin-Gewebekleber kommen deshalb bei vielen verschiedenen Einsatzgebieten zur Anwendung.

Bei tumorchirurgischen Eingriffen insbesondere in der Mund-Kiefer-Gesichtschirurgie sowie im gesamten HNO-Bereich (z.B. Zungenkarzinom-Resektion) kommt es häufig zu schwer beherrschbaren diffusen Blutungen. Die üblicherweise häufig eingesetzte elektrochirurgische Blutstillung durch Elektrokoagulation hinterläßt nach der Koagulation ausgedehnte thermische Gewebenekrosen, die insbesondere in diesen Bereichen äußerst unerwünscht sind.

In der plastisch-ästhetischen Gesichts- und Halschirurgie ("face-lifting") ist die Blutstillung durch Fibrinkleber unverzichtbar, da die Elektrokoagulation wegen der anatomischen Nachbarschaft der Behandlungsstelle zum Verlauf des Gesichtsnerves eine Gefährdung des Gesichtsnerves darstellt und diesen schädigen kann.

Des weiteren ist in der Notfallbehandlung bei zahnärztlich-chirurgischen Eingriffen eine Behandlung mit einem Fibrin-Gewebekleber bei nicht sistierenden Blutungen angezeigt. Dies gilt auch bei Patienten, die wegen eines bestimmten Grundleidens antikoagulativ medikamentös behandelt werden (z.B. Therapie zur Embolieprophylaxe durch Heparine) und trotz des damit verbundenen Risikos der gehemmten Blutgerinnung (verlängerte Blutgerinnung, Thrombozytenfunktionshemmung) operiert werden müssen. In diesem Fall sind deshalb durch die lokale Anwendung eines Fibrin-Gewebeklebers Maßnahmen zu ergreifen, die eine Blutstillung gewährleisten und postoperative Blutungen vermeiden. Dies kann z.B. auch bei Operationen an inneren Organen (z.B. Leber, Milz) erforderlich werden. Der Gewebekleber kann dabei über einen Doppelkatheter von außen endoskopisch zugeführt werden.

Weiterhin ist der Einsatz eines Fibrin-Gewebeklebers bei der Notfallversorgung großflächiger Wunden durch Verbrennungen dritten Grades sowie bei großflächigen Schürfwunden angezeigt.

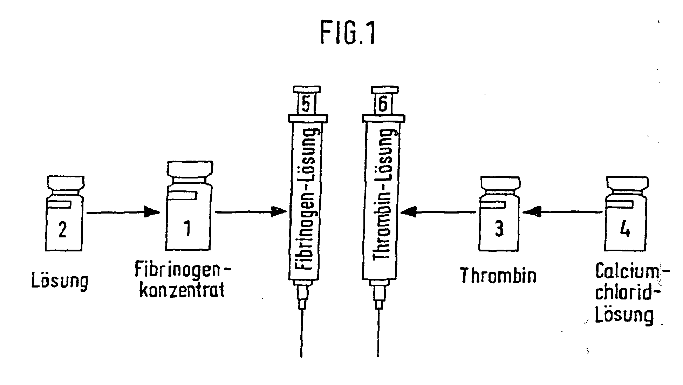

Bei der Darreichung und Anwendung eines Fibrin-Gewebeklebers ist darauf zu achten, daß Fibrinogen und Thrombin erst direkt am Ort der Blutung (d.h. "in der Wunde") zusammengebracht werden, da die einsetzende Gerinnung spontan bei Anwesenheit der Wundflüssigkeit einsetzt. Benachbarte Stellen sind dabei gut abzudecken. Voraussetzung für die Gerinnung ist die freie Beweglichkeit der einzelnen beteiligten Moleküle z.B. in Wasser. Praktisch realisiert wird dies dadurch, daß z.B. die vier verschiedenen Komponenten (Fibrinogen-Faktor XIII-Konzentrat, Lösung für Fibrinogen, Thrombin-Konzentrat, Calcium-Chlorid-Lösung für Thrombin) vor der Anwendung getrennt aufbewahrt werden und erst direkt an der Wunde in gegenseitigen Kontakt gebracht werden. Die Komponenten müssen jeweils steril verpackt werden und in einer geeigneten Form und unter definierten Bedingungen aufbewahrt werden, so daß die Aktivität der einzelnen Proteine bzw. Enzyme durch die Lagerung nicht geschädigt wird. In der Regel wird dies so gelöst, daß die Proteinkonzentrate in gefriergetrockneter Form in kleinen Behältnissen vorliegen. In dieser Form sind sie bei Kühlschrankbedingungen (4 bis 8 °C) für eine bestimmte Zeit und für eine kürzere Zeit auch bei Raumbedingungen (20 °C) lagerstabil. Gefriergetrocknet liegt das Konzentrat jedoch in fester, komprimierter und dadurch unbeweglicher Form, jedoch als löslicher Feststoff vor. Deshalb müssen die Proteinkonzentrate vor der Anwendung wieder vollständig in Lösung gebracht werden, um die gewünschte biochemische Reaktion starten zu können (Fig. 1). Dies darf jedoch erst direkt an der Wunde erfolgen, so daß die Lösungen jeweils vorher getrennt voneinander vorbereitet werden müssen. Vor der Anwendung des Fibrin-Gewebeklebers sollte dann die Wunde möglichst trocken sein, was bei großflächigen, diffusen Blutungen teilweise nur schwer erreichbar ist, um eine gute Fixierung des Gewebeklebers an Ort und Stelle zu ermöglichen. Die beiden Lösungen können jeweils über Injektionsspritzen z.B. im gleichen Volumenverhältnis zugegeben werden. Dabei ist die Fibrinogen-Lösung zuerst auf die Wunde aufzubringen und möglichst sofort mit der Thrombin-Lösung zu überschichten. Die zu klebenden Teile sind dann so lange zu fixieren, bis eine vorläufige Verfestigung eingetreten ist. Alternativ dazu gibt es mechanische Hilfen, z.B. in Form einer Doppelkammer-Injektionsspritze, über die beide Lösungen gleichzeitig auf die Wunde aufgetragen werden können. Weitere technische Hilfsmittel sind z.B. Spray-Tip-Systeme bei großflächigen Wunden, Doppelballonkatheter in der Urologie oder Doppelkatheter zur endoskopischen Anwendung. Die Konzentration der Proteine in beiden Lösungen muß so eingestellt werden, daß Fibrinogen im deutlichen Überschuß gegenüber Thrombin vorliegt. Geeignete Verhältnisse sind gemäß dem Stand der Technik (z.B. 100:1) bekannt.

Dies macht deutlich, daß die Anwendung einerseits einer qualifizierten und konzentrierten Vorbereitung bedarf, die in Notfallsituationen teilweise nicht immer sichergestellt werden kann. Andererseits ist die Anwendung durch die umständliche und manuelle Handhabung des 2-Spritzen-Systems ebenfalls eingeschränkt.

Aus der WO 97/44015 ist eine sprühgetrocknete Gewebekleber-Formulierung bekannt. Diese Mikropartikel weisen jedoch eine sphärische Oberfläche auf und besitzen eine Korngröße von max. 20 µm. Dadurch ist dieses Produkt nicht rieselfähig und schlecht zu dosieren. Es hat sich gezeigt, daß dieses Produkt nicht nur bei seiner Anwendung staubt sondern auch eine schlechte Löslichkeit aufweist.

Aus dem Lehrbuch der Pharmazeutischen Technologie 5 Auflage 1984 Seiten 156-166 ist bekannt daß Granulat durch Wirkelschich- und Sprühtrocknung erhalten werden können.

Aufgabe der vorliegenden Erfindung ist es deshalb, eine Fibrin-Gewebekleber-Formulierung anzugeben, die in der Handhabung, Dosierung und Anwendung einfach ist und über längere Zeit problemlos aufbewahrt werden kann, so daß die Einsatzmöglichkeiten einer derartigen Fibrin-Gewebekleber-Formulierung gegenüber dem Stand der Technik deutlich erweitert werden.

Aufgabe der Erfindung ist es gleichfalls ein entsprechendes Verfahren zur Herstellung einer derartigen Fibrin-Gewebekleber-Formulierung anzugeben.

Die Aufgabe wird in bezug auf die Formulierung durch die kennzeichnenden Merkmale des Anspruches 1 und in bezug auf das Verfahren durch die kennzeichnenden Merkmale des Patentanspruches 13 gelöst.

Die Unteransprüche zeigen vorteilhafte Weiterbildungen auf.

Erfindungsgemäß wird somit vorgeschlagen, daß die Fibrin-Gewebekleber-Formulierung in fester rieselfähiger Form als Gemisch der verschiedenen Proteinkonzentrate vorliegt, wobei die Granulatgröße im Bereich zwischen 40 und 200 µm liegt und somit problemlos in der Handhabung und in der Anwendung ist. Erfindungswesentlich ist dabei, daß die in der Formulierung enthaltenen Granulate durch Trocknung der Proteinlösung in einer Wirbelschicht hergestellt werden, da es sich überraschenderweise gezeigt hat, daß mit diesen Verfahren eine so schonende Trocknung der Proteinlösungen bzw. Suspensionen möglich ist, daß sich ihre funktionalen Eigenschaften nicht ändern. Ein weiterer Vorteil ist darin zu sehen, daß das Granulat in rieselfähiger Form vorliegt, so daß eine exakte Dosierung möglich ist.

Die Fibrin-Gewebekleber-Formulierung nach der Erfindung weist damit weitreichende Vorteile gegenüber dem Stand der Technik auf. Die Erfindung zeichnet sich insbesondere dadurch aus, daß
- das Gemisch (= der Fibrin-Gewebekleber) nicht reagiert (d.h. die Gerinnung auslöst), solange es in dieser festen Form vorliegt;
- das Gemisch (= der Fibrin-Gewebekleber) in fester und jedoch gleichzeitig pulver- bzw. granulatförmigen, dadurch rieselfähigen und staubfreien Form vorliegt, wodurch das Gemisch direkt auf die zu versorgende Wunde aufgetragen werden kann, ohne daß von der Anwendung die Protein-Komponenten (Fibrinogen-Faktor XIII-Konzentrat und Thrombin-Konzentrat) in Lösung gebracht werden müssen;
- das Gemisch (= der Fibrin-Gewebekleber) sich in der Wundflüssigkeit gut, vollständig und schnell löst;
- das Gemisch (= der Fibrin-Gewebekleber), nachdem bzw. während es sich in der Wundflüssigkeit gelöst hat bzw. löst, die biochemische Reaktion der Blutgerinnung auslöst und eine sich fixierende feste Schicht bildet und damit eine geeignete Wundversorgung darstellt;
- durch die Möglichkeit, die Partikelgröße vergleichsweise einfach variieren zu können, neue Anwendungsmöglichkeiten resultieren. Beispielsweise in der Form, daß es möglich wird, durch Variation der Partikelgröße bei der Dosierung den Wundkontakt entweder stark lokalisieren zu können (bei homogen verteilten, größeren Partikeln) oder auch großflächig in einer dünnen Pulverschicht (z.B. durch Spray-Systeme bei feinem Granulat) zu ermöglichen;
- sich im Gemisch als Granulatmischung unterschiedliche Mischungsverhältnisse beider Komponenten leicht einstellen lassen und so die Eigenschaften des Fibrin-Gewebeklebers (Löslichkeit, Einsetzen der Gerinnung) gezielt eingestellt werden können;
- durch die Tatsache, daß sich Pulver sehr homogen vermischen läßt, der "content uniformity" gut sicherstellen läßt, auch wenn ein breites Partikelgrößenspektrum vorliegt (d.h. das unabhängig von Partikeleigenschaften wie Korngröße Dichte, und andere immer das gewünschte Mischungsverhältnis besteht).

Die erfindungsgemäße Fibrin-Gewebekleber-Formulierung enthält bevorzugt noch ein Calciumsalz z.B. CaCl₂ und kann dabei so aufgebaut sein, daß entweder die einzelnen Proteinlösungen bzw. Suspensionen, d.h. die Fibrinogen-Faktor XIII-Lösung bzw. Suspension und die Thrombin/CaCl₂-Lösung bzw. Suspension separat getrokknet und dann die getrockneten Granulate gemischt werden, oder daß bei der Trocknung der Proteinlösung zuerst das Fibrinogen getrocknet und dann auf dieses so hergestellte Granulat das Thrombin aufgebracht wird. Auch ist ein Aufbau möglich, bei dem das Thrombin den Kern bildet.

Bei der Fibrin-Gewebekleber-Formulierung nach der Erfindung ist weiterhin hervorzuheben, daß diese je nach Anwendungsfall eingestellt werden kann. So kann in der Gewebekleber-Formulierung zum einen das Mischungsverhältnis von Fibrinogen zu Thrombin gezielt je nach Anwendungsfall ausgewählt werden, zum anderen ist auch eine Steuerung der Partikelgröße möglich.

Bei der Fibrin-Gewebekleber-Formulierung bei der jeweils zuerst separate Granulate der jeweiligen Proteine hergestellt und diese dann vermischt werden, ist es auch möglich, daß das Granulat aus einem Kern, aus einem Trägermaterial und einer darauf aufgebrachten Proteinschicht besteht. Das Trägermaterial kann z.B. aus wasserlöslichen Zuckern und/oder Zuckeraustauschstoffen und/oder biologischen Transportsubstanzen bestehen. Beispiele sind Mannitol oder Serum-Albumin.

Fibrin-Gewebekleber-Formulierungen mit einem Kern, d.h. mit einem Trägermaterial sind auch bei den Mischgranulaten bevorzugt. In diesem Fall besteht dann das Granulat aus einem Kern, z.B. wieder aus Mannitol, auf dem dann eine Fibrinogenschicht aufgebracht ist, über der dann die Thrombinschicht angeordnet ist. Diese Mischgranulate haben demnach einen dreischichtigen Aufbau. Selbstverständlich ist es gemäß der vorliegenden Erfindung auch möglich, daß diese Mischgranulate ohne Kern hergestellt werden. Bei der Ausführungsform mit den Mischgranulaten ist es weiterhin bevorzugt, wenn zwischen der Fibrinogenschicht und der Thrombinschicht eine Sperrschicht angeordnet ist. Diese Sperrschicht muß zum einen die Fibrinogenschicht von der Thrombinschicht trennen und muß zum anderen aber auch gut wasserlöslich sein. Materialien für diese Sperrschicht müssen deshalb die beiden vorstehend genannten Kriterien erfüllen. Beispiele hierfür sind niedermolekulare Polyvenylpyrrolidone oder auch Cellulosederivate oder auch Kohlehydrate, z.B. Dextrosederivate.

Die Erfindung betrifft weiterhin ein Verfahren zur Herstellung der vorstehend beschriebenen Fibrin-Gewebekleber-Formulierung.

Erfindungsgemäß wird vorgeschlagen, die im Fibrin-Gewebekleber typischerweise vorkommenden Proteine Fibrinogen, Thrombin, Faktor XIII sowie Calciumsalz in einer Wirbelschichtapparatur, schonend getrocknet werden, so daß dadurch ein rieselfähiger, granulatförmiger Feststoff entsteht. Eine geeignete Vorrichtung hierfür ist in der DE 44 41 167 beschrieben. Auf diesen Offenbarungsgehalt wird deshalb Bezug genommen.

Bevorzugt wird das Verfahren so ausgeführt, daß das Fluidisationsgas durch die Wirbelschichtkammer von unten nach oben geführt wird und die zu trocknende Flüssigkeit (Lösung oder Suspension) von oben (Top-Spray), von unten (Bottom-Spray) oder auch seitlich (Rotor-Wirbelschicht) über ein Sprühsystem eingesprüht wird. Das Fluidisationsgas hat gleichzeitig die Aufgabe, in der Wirbelkammer vorliegendes Produkt zu verwirbeln, die zum Verdunsten der Sprühflüssigkeit (Wasser oder organisches Lösungsmittel) benötigte Wärme dem Sprühstrahl oder dem feuchten Produkt zuzuführen und gleichzeitig die verdunstete Flüssigkeitsmenge aufzunehmen und abzutransportieren. Der Austrag des getrockneten Produktes wird einerseits durch die Wahl einer geeigneten Fluidisationsgeschwindigkeit (kleiner als die rechnerisch und experimentell ermittelbare sog. Austragsgeschwindigkeit für das Produkt), andererseits auch durch einen im oberen Bereich der Wirbelkammer vorhandenen und regelmäßig abreinigbaren Produktrückhaltefilter oder auch durch einen anderen, aus dem Stand der Technik bekannten Produktabscheider (wie z.B. ein Zyklonabscheider) verhindert.

Vorgegangen werden kann dabei z.B. derart, daß in der Wirbelkammer das Trägermaterial vorgelegt wird auf das dann z.B. aus wäßriger Protein-Lösung bzw. Suspension die Lösung/Suspension aufgesprüht wird. Die im Sprühkegel fein zerstäubten Flüssigkeitströpfchen treffen dabei auf das aufgewirbelte pulverige Trägermaterial und trocknen dort aufgrund der für Wirbelschichtverfahren idealen Wärme- und Stoffübergangsverhältnisse, die im wesentlich eine Folge der sehr großen spezifischen Partikeloberflächen des verwirbelten Produktes sind. Auf dem Träger lagern sich dann die in der Sprühflüssigkeit vorhandenen Proteine als Feststoff infolge adsorptiver Kräfte an. Der Träger ist idealerweise so beschaffen, daß er einerseits inert gegenüber den Proteinen ist (d.h. es darf zu keiner Wechselwirkung mit dem Proteinstrukturen kommen, was die funktionellen Eigenschaften bleibend ändern würde) und daß gleichzeitig die Löslichkeit der Proteine in Wasser, Wundflüssigkeit oder physiologischer Kochsalzlösung eingeschränkt oder verhindert wird. In Frage kommen deshalb z.B. gut wasserlösliche Zucker (z.B. Mannitol) oder auch andere, gemäß dem Stand der Technik als gut wasserlösliche Trägerstoffe bekannte Substanzen. Diese müssen jedoch, aufgrund der sehr spezifischen Eigenschaften der Proteine, auf deren Eignung einzeln bewertet werden. Als Träger auch geeignet sind Substanzen, die bereits im biologischen System als Transportsysteme fungieren und die gleichzeitig deshalb eingesetzt werden können, da sie in den natürlichen, biologischen Systemen neben den gewünschten Proteinen des Fibrin-Gewebeklebers vorliegen. Als Beispiel kann hierfür Serum-Albumin humanen Ursprungs oder in rekombinanter Form genannt werden.

Während des Sprühens kommt es aufgrund der im Partikel langsam zunehmenden Produktfeuchte zur Ausbildung von Agglomeraten oder Granulaten und dadurch zu einer Zunahme der Partikelgröße. Um die gute Wasserlöslichkeit zu erhalten, kann es vorteilhaft sein, amorphe Granulatstrukturen mit den daraus folgenden großen spezifischen Oberflächen zu erzeugen. Geeignete Prozeßbedingungen (Variation des Sprühdruckes, Sprührate, Produkttemperatur und Zulufttemperatur, Feststoff-Konzentration der eingesetzten Sprühlösung), um diese Strukturen definiert und reproduzierbar zu erzeugen, sind gemäß dem Stand der Technik von Wirbelschichtverfahren bekannt. Durch Zugabe gemäß dem Stand der Technik bekannter wasserlöslicher Bindemittel (z.B. Cellulose-Derivate) kann die Partikelgröße in der Größe und in der Korngrößenverteilung variiert werden (Schäfer, T.: Worts, O.: Control of fluidized bed granulation. V. Factors affecting granule gowth. Arch. Pharm. Chemie. Sci. Ed. 6, 1978, 69-82).

Mit der exakten Einstellung einer bestimmten Partikelgröße können die Forderungen "Rieselfähigkeit" (und damit auch Dosierbarkeit), "Löslichkeit", "Staubfreiheit" und "Mischbarkeit" gut eingestellt und auch bewußt variiert werden. So ist es vorteilhaft, durch eine möglichst feine und kleine Partikelgröße eine großflächige, feindisperse Anwendung des Fibrin-Gewebeklebers zu ermöglichen. Gleichzeitig kann durch größere Partikel mit enger Partikelgrößenverteilung eine lokal stark begrenzte, gezielte Dosierung des Fibrin-Gewebeklebers möglich werden. Ein weiterer Freiheitsgrad für die Anwendung eines festen, rieselfähigen Fibrin-Gewebeklebers kann z.B. die Löslichkeit des Granulats darstellen. Damit kann z.B. eine maximal schnelle Löslichkeit oder eine verzögerte Löslichkeit und eine damit auch verzögert oder langsamer einsetzende Gerinnung eingestellt werden. Diese langsame oder verzögerte Gerinnung kann z.B. bei plastisch-ästhetischen Gesichtsoperationen zusätzliche Möglichkeiten zur zusätzlichen Manipulation oder Änderung operativer Eingriffe schaffen. Die Löslichkeit läßt sich sowohl über die Partikelgröße, die Partikelstruktur und über zusätzliche Substanzen, die die inneren Bindungskräfte erhöhen oder erniedrigen, beeinflussen.

Bei der Wahl der Prozeßbedingungen muß weiterhin primär darauf geachtet werden, daß die relevanten Proteine nicht geschädigt werden (z.B. durch hohe Temperaturen). Geeignete Zulufttemperaturen liegen z.B. zwischen 15 und 100 °C; für die Produkttemperatur bevorzugt jedoch kleiner 50 oder 37 °C. Berücksichtigt werden muß dabei, daß eine mögliche Inaktivierung immer im Zusammenhang mit einer bestimmten Feuchte betrachtet werden muß, d.h. die Temperaturstabilität nimmt mit abnehmender Produktfeuchte im Feststoff zu, so daß gegen Ende der Trocknung auch höhere Temperaturen akzeptabel sein können.

Die Trocknung muß bis zu einer Restfeuchte erfolgen, die so klein ist, daß je nach den gewählten Lagerbedingungen keine Aktivitätsverluste beobachtet werden oder daß es bereits zu einem selbsttätigen Ablaufen der Gerinnung kommt. Geeignete Lagerbedingungen sind: Kühllagerung bei 4 bis 8 °C oder Raumbedingungen (20 °C). Das Granulat kann zusätzlich in einer schützenden Atmosphäre (z.B. Stickstoff oder Kohlendioxid) und z.B. unter Lichtausschluß eingeschlossen sein. Mögliche Restfeuchten können dann z.B. zwischen 0,1 - 5 % Wassergehalt liegen.

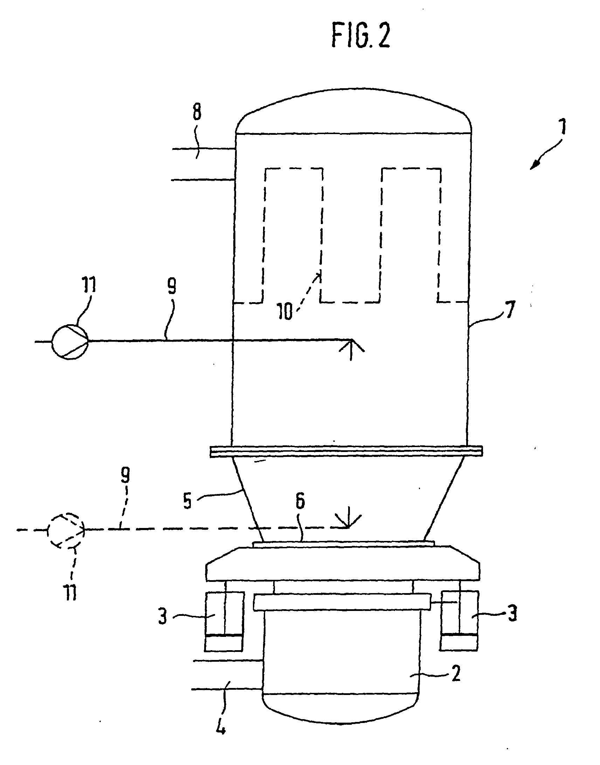

Die Erfindung wird nachfolgend durch Beispiele und die Figuren 1 und 2 näher erläutert.

Fig. 1 zeigt schematisch das 2-Spritzenmodell, Fig. 2 zeigt eine Wirbelschichtanlage zur Durchführung des Verfahrens.

Allgemeine Vorschriften zur Herstellung des Granulats:
(1) Auf vorgelegtes Mannitol (mit Partikelgröße 50-100 µm) wird das Fibrinogen-Konzentrat (zusammen mit dem Faktor XIII) aus wäßriger Lösung aufgesprüht. Das Verhältnis Trägermaterial zu Proteinmenge kann z.B. in einem Bereich von 1:1 bis 100:1 variiert werden und liegt bevorzugt in einem Bereich von 1:1 bis 10:1. Getrocknet wird bis zur geeigneten Restfeuchte, die Produkttemperatur überschreitet während des Sprühens und des anschließenden Nachtrocknens die Temperatur von 35 °C nicht.
(2) Anschließend wird auf gleiche Weise auf ebenfalls vorgelegtes Mannitol (mit Partikelgröße 50 - 100 µm) das Thrombin-Konzentrat aus wäßriger Lösung mit einer definierten Menge Calcium-Chlorid aufgesprüht. Da Thrombin im Fibrin-Gewebekleber mengenmäßig den deutlich kleineren Anteil hat, liegt das Verhältnis Trägermaterial zu Proteinmenge für Thrombin z.B. in einem Bereich von 50:1 bis 1000:1 und bevorzugt im Bereich 50:1 bis 200:1. Im Anschluß an das Sprühen wird ebenfalls bis zu einer geeigneten Restfeuchte unter Einhaltung einer maximalen Produkttemperatur von 35 °C getrocknet. Beide erhaltenen Granulate werden anschließend vermischt und können dann direkt als Mischung auf die Wunde aufgetragen werden. Das Mischungsverhältnis orientiert sich an dem, gemäß dem Stand der Technik vorgegebenen, Verhältnis von Fibrinogen zu Thrombin, wie dieses auch bei den bisher bekannten flüssigen Darreichungsformen eingestellt wird. Darüber hinaus sind jedoch auch andere Mischungsverhältnisse Fibrinogen-Granulat zu Thrombin-Granulat gut und leicht einstellbar (anders als bei Lösungen, wo sich die Volumenverhältnisse an der Löslichkeit orientieren müssen). Somit kann durch definierte, homogene Mischungen die Wirkung des Fibrin-Gewebeklebers hinsichtlich Einsetzen der Gerinnung, Beginn einer irreparablen Verfestigung oder auch Festigkeit des vollständig geronnenen Klebers einfach und gezielt beeinflußt werden.

Alternativ dazu kann auch gemäß folgendem Prozeßablauf ein Fibrin-Gewebekleber hergestellt werden:
(3) Durchführung der Trocknung von Fibrinogen wie unter (1) beschrieben (auf Trägermaterial).
(4) Auf das getrocknete Granulat wird Thrombin aus einer organischen Suspension (geeignet ist z.B. Isopropanol) zusammen mit Calcium-Chlorid aufgesprüht. Thrombin (und auch Fibrinogen) ist in Isopropanol stabil, wird chemisch dadurch nicht verändert, löst sich jedoch nicht in Isopropanol. Thrombin lagert sich somit auf das mit Fibrinogen beladene Granulat an. Durch die Abwesenheit von Wasser kommt es nicht zu einer vorzeitigen Gerinnung z.B. bereits auf dem Granulat während der Sprühgranulation. Das Mischgranulat, bestehend aus Träger, Fibrinogen-Faktor XIII und Thrombin, kann direkt auf der Wunde angewandt werden. Anteile Fibrinogen zu Thrombin entsprechen wieder dem aus dem Stand der Technik bekannten Verhältnis. Die Löslichkeit, und damit verbunden auch die Gerinnung, wird bei diesem Mischgranulat, insbesondere auch durch die Abwesenheit einer erheblichen Trägermaterialmenge, die sich bei der Anwendung nicht erst lösen muß, erhöht.
(5) Um ein direktes Aufsprühen von Thrombin-haltiger, wäßriger Lösung (+ CaCl₂) auf vorgelegtes Fibrinogen-Granulat (hergestellt gemäß (1)) zu ermöglichen, kann als innere Barriere auf das Fibrinogen-Granulat z.B. eine gut wasserlösliche Sperrschicht als räumliche Trennung von Fibrinogen und Thrombin aufgebracht werden. Für diese Sperrschicht gilt, daß zum einen beide Wirkstoffe dadurch chemisch nicht verändert werden dürfen, daß die Sperrschicht gut wasserlöslich ist und daß sie eine wirksame Trennung von Fibrinogen und Thrombin während des Sprühens und Granulierens und auch in der endgültigen, lagerstabilen, festen, getrockneten Form darstellt. Dafür geeignet sind z.B. niedermolekulare, Polyvinylpyrrolidon- oder auch Cellulose-Derivat-Lösungen oder auch Kohlehydrate (z.B. Dextrose-Derivate). Für das so hergestellte Produkt ist die gleiche Charakteristik bezüglich Löslichkeit und Gerinnung zu erwarten wie für das gemäß (4) produzierte Granulat.

Daneben sind auch Prozeßvarianten ohne ein zusätzlich vorgelegtes Trägermaterial möglich:
(6) Durch Einsprühen aus wäßriger Fibrinogen-Lösung oder aus isopropanolischer (bzw. organischer) Suspension in eine leere Anlage werden in-situ Granulatkeime bzw. fein verteilte Partikel erzeugt, die als Starterkerne für eine weitere Granulation dienen können. Die dafür zu verwendende Anlage kann z.B. ein Sprühturm oder auch eine Wirbelschichtanlage mit ausreichend freier Flugstrecke für die versprühten Flüssigkeitströpfchen sein. Bei Einhaltung geeigneter Prozeßbedingungen können die versprühten Flüssigkeitströpfchen entsprechend den Verhältnissen eines Sprühtrockners (jedoch mit reduzierten Trocknungstemperaturen) in einer Wirbelschichtanlage getrocknet werden, bevor sie z.B. im noch feuchten Zustand die Behälterwand berühren und dort kleben bleiben. Diese so erzeugten feinen Partikel werden durch das Fluidisationsgas in Bewegung und in der Schwebe gehalten und kommen so mit dem Sprühnebel der weiterhin eingesprühten Flüssigkeit in Kontakt und beginnen dann zu granulieren. Auf diese Weise kann, insbesondere durch sehr vorsichtige Fahrweise des Prozesses während des Anfahrens des Prozesses, in der ursprünglich leeren Anlage ein definiertes Granulatwachstum generiert werden. Dies kann z.B. durch Zugabe bekannter Bindemittel unterstützt werden. Durch Kombination mit einem klassierenden Granulataustrag (z.B. über einen Zick-Zack-Sichter und klassierendem Luftstrom) besteht die Möglichkeit, Granulat mit einer definierten Partikelgröße in der Anlage zu erzeugen und den Prozeß sogar in einer kontinuierlichen oder quasi-kontinuierlichen Fahrweise zu betreiben.
(7) Auf das gemäß (6) erzeugte Fibrinogen-Konzentrat-Granulat kann direkt wie unter (4) oder (5) beschrieben, Thrombin mit oder ohne eine zusätzliche Sperr-(bzw. Coating-)schicht aufgebracht werden.

Gemäß dem Stand der Technik können bzw. müssen die Herstellungsvarianten (1)-(7) für den Fibrin-Gewebekleber mit geeigneten Verfahren zum Inaktivieren von Viren kombiniert werden. Dies kann entweder so erfolgen, daß die Proteinkonzentrate vor der Trocknung mit bekannten Inaktivierungs-Verfahren (z.B. Pasteurisieren oder Solvent/Detergent-Verfahren) behandelt werden, oder daß das getrocknete Granulat, wie aus DE 44 41 167 bekannt, direkt in der Wirbelschicht gegen Ende oder nach der eigentlichen Sprühgranulation oder Trocknung derartig wärmebehandelt wird, daß die Viren entsprechend inaktiviert werden. Dieser Behandlungsschritt muß jedoch so durchgeführt werden, daß die funktionellen Eigenschaften der Proteine erhalten bleiben.

Fig. 1 zeigt die schematische Darstellung der erforderlichen Vorbereitung der verschiedenen Komponenten eines Fibrin-Gewebeklebers vor der Anwendung und Möglichkeiten zur Verabreichung nach dem Stand der Technik.
- 1: Fibrinogen-Faktor XIII-Konzentrat
- 2: Lösung (z.B. physiologische Kochsalzlösung)
- 3: Thrombin-Konzentrat
- 4: Calcium-Chlorid-Lösung

Die Komponenten 1-4 sind sterilverpackt. In der Regel werden die Lösungen aus den Komponenten 2 und 4 mittels Vakuum in die Flaschen 1 und 3 gebracht. Nachdem sich in den Behältnissen 1 und 3 eine vollständige Lösung (ohne Trübung) gebildet hat, können die Lösung in Sterilspritzen (5) und (6) aufgezogen werden und an bzw. in einer Wunde zur Verabreichung kommen. Die eingesetzten Mengen liegen typischerweise im ml-Bereich.

Fig. 2 zeigt eine mögliche Ausführungsform einer Wirbelschichtanlage zur Herstellung des Granulates.
- 1: Wirbelschichtanlage
- 2: Unterteil
- 3: Anpreßvorrichtung (z.B. Hydraulikzylinder)
- 4: Zuluftkanal
- 5: Materialbehälter
- 6: Anströmboden (Gasverteiler)
- 7: Filtergehäuse (Entspannungszone)
- 8: Abluftkanal
- 9: Sprühkanal mit Sprühdüse (Top-Spray und Bottom-Spray-Position)
- 10: Produktrückhaltefilter
- 11: Sprühpumpe

Mittels eines Fluidisationsgases wird Produkt, Pulver oder Granulat in einer Wirbelschichtanlage 1 verwirbelt. Das Fluidisationsgas wird dabei durch die Wirbelschichtanlage 1 von unten nach oben z.B. mittels eines nicht dargestellten Ventilators durchgeführt. Die Aufgabe des Fluidisationsgases ist somit die Verwirbelung des zu behandelnden Gutes, die konvektive Wärmezufuhr an das Produkt bzw. an einen Sprühnebel und der Abtransport der verdunsteten Flüssigkeitsmenge während der Trocknung. Der Einlaß des Fluidisationsgases erfolgt über den im Unterteil 2 angebrachten Zuluftkanal 4. Die gleichmäßige Gasverteilung über dem Querschnitt des Reaktionsraumes erfolgt über einen Anströmboden 6, der gleichzeitig den Materialbehälter 5 vom Unterteil 2 trennt. Im Filtergehäuse 7 sind im oberen Bereich technische Hilfsmittel (z.B. Produktrückhaltefilter) zur Rückhaltung von feinkörnigem Produkt angebracht, die sicherstellen, daß kein Produktaustrag in den ebenfalls im oberen Bereich der Wirbelschichtanlage angebrachten Abluftkanal 8 erfolgen kann. Flüssiges Produkt (Lösung oder Suspension) kann über einen Sprühkanal mit Sprühdüse 9 und mittels einer Sprühpumpe 11 aus einer nicht dargestellten Vorlage in die Wirbelschichtanlage 1 entweder von oben (Top-Spray-Position, mit durchgezogener Linie dargestellt) oder von unten (Bottom-Spray-Position, gestrichelt dargestellt) eingesprüht werden. Der dadurch entstehende Sprühkegel trifft entweder auf bereits im Materialbehälter 5 vorgelegtes Produkt und trocknet dort auf der daraus resultierenden Partikeloberfläche oder wird analog zu Sprühtrocknungsbedingungen im Reaktionsraum direkt getrocknet und bildet so Pulver bzw. feinverteiltes Granulat. Durch eine Messung der Produkttemperatur während des Wirbelschichtprozesses und einer darauf basierenden Prozeßsteuerung kann eine produktschonende Trocknung eingehalten werden. Die Temperatur des Fluidisationsgases ist dabei selbstverständlich nach dem zu behandelnden Gut ausgewählt und kann z.B. in einem Bereich von 15 bis 100 °C liegen. Die resultierende Produkttemperatur ist niedriger und kann bevorzugt kleiner 50 °C oder besser kleiner 37 °C während der Trocknung bzw. Sprühgranulation gehalten werden.

## Patentansprüche

1. Fibrin-Gewebekleber-Formulierung enthaltend Thrombin, Fibrinogen und Faktor XIII
**dadurch gekennzeichnet,**
**daß** das Thrombin und Fibrinogen mit Faktor XIII als Gemisch in rieselfähiger fester Granulatform vorliegt, wobei das Granulat durch Trocknung der Proteinlösungen oder Suspensionen in einer Wirbelschichtapparatur erhalten worden ist, und daß das Granulat eine Partikelgröße von 40-200 µm aufweist.

2. Fibrin-Gewebekleber-Formulierung nach Anspruch 1, **dadurch gekennzeichnet, daß** das Gemisch aus separat getrockneten Thrombin- und Fibrinogengranulaten besteht.

3. Fibrin-Gewebekleber-Formulierung nach Anspruch 1 oder 2, **dadurch gekennzeichnet, daß** die Thrombinund/oder Fibrinogengranulate einen Kern als Träger aufweisen.

4. Fibrin-Gewebekleber-Formulierung nach Anspruch 3, **dadurch gekennzeichnet, daß** der Träger ausgewählt ist aus wasserlöslichen Zuckern und/oder Zukkeraustauschstoffen und/oder biologischen Transportsubstanzen.

5. Fibrin-Gewebekleber-Formulierung nach Anspruch 1, **dadurch gekennzeichnet, daß** das Gemisch ein Mischgranulat ist, bei dem Thrombin die äußere Schicht und das Fibrinogen den inneren Kern bildet.

6. Fibrin-Gewebekleber-Formulierung nach Anspruch 5, **dadurch gekennzeichnet, daß** das Mischgranulat aus einem Kern als Träger einer darauf angeordneten Fibrinogenschicht und einer äußeren Schicht aus Thrombin besteht.

7. Fibrin-Gewebekleber-Formulierung nach Anspruch 5 oder 6, **dadurch gekennzeichnet, daß** zwischen der Fibrinogen- und der äußeren Thrombinschicht eine Sperrschicht vorhanden ist.

8. Fibrin-Gewebekleber-Formulierung nach mindestens einem der Ansprüche 5 bis 7, **dadurch gekennzeichnet, daß** die Sperrschicht aus niedermolekularem Polyvinylpyrrolidon oder Cellulosederivatlösungen oder Kohlehydratlösungen durch Trocknung dieser Lösungen hergestellt worden ist.

9. Fibrin-Gewebekleber-Formulierung nach mindestens einem der Ansprüche 1 bis 8, **dadurch gekennzeichnet, daß** das Verhältnis von Thrombin zu Fibrinogen mit Faktor XIII im Bereich von 1:10 bis 1:1000, bevorzugt von 1:50 bis 1:200 liegt.

10. Fibrin-Gewebekleber-Formulierung nach mindesten einem der Ansprüche 1 bis 9, **dadurch gekennzeichnet, daß** die Thrombingranulate und/oder Fibrinogengranulate und/oder Mischgranulate mit einer äußeren Sperrschicht versehen sind.

11. Fibrin-Gewebekleber-Formulierung nach mindestens einem der Ansprüche 1 bis 10, **dadurch gekennzeichnet, daß** die Thrombin- und Fibrinogenproteine durch gentechnologische oder biotechnologische Verfahren rekombinant hergestellt worden sind.

12. Fibrin-Gewebekleber-Formulierung nach mindestens einem der Ansprüche 1 bis 11, **dadurch gekennzeichnet, daß** sie ein Calciumsalz enthält.

13. Verfahren zur Herstellung einer Fibrin-Gewebekleber-Formulierung nach mindestens einem der Ansprüche 1 bis 12, **dadurch gekennzeichnet, daß** die Proteinlösungen oder Suspensionen von Fibrinogen mit Faktor XIII und Thrombin in eine Wirbelschichtkammer eingesprüht und mittels eines Fluidationsgases getrocknet werden, wobei die maximale Produkttemperatur 50 °C nicht überschreitet.

14. Verfahren nach Anspruch 13, **dadurch gekennzeichnet, daß** in einem ersten Schritt ein Fibrinogenkonzentrat mit Faktor XIII aus wäßriger Lösung in die Wirbelschichtkammer eingesprüht und getrocknet sowie isoliert wird und daß dann in einem zweiten Schritt das Thrombinkonzentrat aus wäßriger Lösung in die Wirbelschichtkammer eingesprüht, getrocknet und isoliert wird und daß dann anschließend beide erhaltenen Granulate vermischt werden.

15. Verfahren zur Herstellung einer Fibrin-Gewebekleber-Formulierung nach Patentanspruch 13, **dadurch gekennzeichnet, daß** in einem ersten Schritt ein Fibrinogenkonzentrat aus wäßriger Lösung in die Wirbelschichtkammer eingesprüht und getrocknet wird und daß dann auf dieses getrocknete Granulat Thrombin aus einer organischen Suspension aufgesprüht wird.

16. Verfahren zur Herstellung einer Fibrin-Gewebekleber-Formulierung nach mindestens einem der Ansprüche 13 bis 15, **dadurch gekennzeichnet, daß** die Proteinlösungen auf ein vorgelegtes Trägermaterial aufgesprüht werden.

## Claims

1. Fibrin tissue adhesive formulation containing thrombin, fibrinogen and factor XIII **characterised in that** the thrombin and fibrinogen with factor XIII is present as a mixture in pourable solid granule form, wherein the granules has been obtained by drying the protein solutions or suspensions in a fluidised bed apparatus and the granules have a particle size from 40 - 200 µm.

2. Fibrin tissue adhesive formulation according to claim 1, **characterised in that** the mixture consists of separately dried thrombin and fibrinogen granules.

3. Fibrin tissue adhesive formulation according to one of claims 1 or 2, **characterised in that** the thrombin and/or fibrinogen granules have a core as carrier.

4. Fibrin tissue adhesive formulation according to claim 3, **characterised in that** the carrier is selected from water-soluble sugars and/or sugar substitutes and/or biological transport substances.

5. Fibrin tissue adhesive formulation according to claim 1, **characterised in that** the mixture is mixed granules, in which thrombin forms the outer layer and the fibrinogen the inner core.

6. Fibrin tissue adhesive formulation according to claim 5, **characterised in that** the mixed granules consist of a core as carrier for a fibrinogen layer arranged thereon and an outer layer of thrombin.

7. Fibrin tissue adhesive formulation according to claim 5 or 6, **characterised in that** a carrier layer is present between the fibrinogen layer and the outer thrombin layer.

8. Fibrin tissue adhesive formulation according to at least one of claims 5 to 7, **characterised in that t**he barrier layer has been produced from low-molecular polyvinylpyrrolidone or cellulose derivative solutions or carbohydrate solutions by drying these solutions.

9. Fibrin tissue adhesive formulation according to at least one of claims 1 to 8, **characterised in that** the ration of thrombin to fibrinogen with factor XIII lies in the range from 1:10 to 1:1000, preferably 1:50 to 1:200.

10. Fibrin tissue adhesive formulation according to at least one of claims 1 to 9 , **characterised in that** the thrombin granules and/or fibrinogen granules and/or mixed granules are provided with an outer barrier layer.

11. Fibrin tissue adhesive formulation according to at least one of claims 1 to 10, **characterised in that** the thrombin and fibrinogen proteins have been produced recombinantly by genetic engineering or biotechnological processes.

12. Fibrin tissue adhesive formulation according to at least one of claims 1 to 11, **characterised in that** it contains a calcium salt.

13. Process for producing a fibrin tissue adhesive formulation according to at least one of claims 1 to 12, **characterised in that** the protein solutions or suspensions of fibrinogen with factor XIII thrombin are sprayed into a fluidised bed chamber and dried by means of a fluidisation gas, wherein the maximum product temperature does not exceed 50 °C.

14. Process according to claim 13, **characterised in that** in a first step a fibrinogen concentrate with factor XIII is sprayed into the fluidised bed chamber from aqueous solution and dried and isolated, and **in that** then in a second step the thrombin concentrate is sprayed into the fluidised bed chamber from aqueous solution, dried and isolated, and **in that** then subsequently both granules obtained are mixed.

15. Process for producing a fibrin tissue adhesive formulation according to patent claim 13, **characterised in that** in a first step a fibrinogen concentrate is sprayed into the fluidised bed chamber from aqueous solution and dried, and **in that** then thrombin is sprayed onto these dried granules from an organic suspension.

16. Process for producing a fibrin tissue adhesive formulation according to at least one of claims 13 to 15, **characterised in that** the protein solutions are sprayed onto a provided carrier material.

## Revendications

1. Formulation de colle tissulaire à base de fibrine comprenant de la thrombine, du fibrinogène et le facteur XIII, **caractérisée en ce que** la thrombine et le fibrinogène avec le facteur XIII sont présents comme un mélange sous la forme de granulats solides coulants, le granulat ayant été obtenu par séchage des solutions de protéines ou des suspensions dans un dispositif à lit fluidisé, et **en ce que** le granulat présente une granulométrie comprise entre 40 à 200 µm.

2. Formulation de colle tissulaire à base de fibrine, selon la revendication 1, **caractérisée en ce que** le mélange est constitué de granulats de thrombine et de fibrinogènes séchés séparément.

3. Formulation de colle tissulaire à base de fibrine selon la revendication 1 ou 2, **caractérisée en ce que** les granulats de thrombine et/ou de fibrinogènes présentent un noyau servant de support.

4. Formulation de colle tissulaire à base de fibrine selon la revendication 3, **caractérisée en ce que** le support est choisi parmi des sucres solubles dans l'eau et/ou des produits de substitution de sucre et/ou des substances de transport biologiques.

5. Formulation de colle tissulaire à base de fibrine selon la revendication 1, **caractérisée en ce que** le mélange est un granulat mixte, la thrombine constituant la couche externe et le fibrinogène le noyau interne.

6. Formulation de colle tissulaire à base de fibrine selon la revendication 5, **caractérisée en ce qui** le granulat mixte est constitué d'un noyau servant de support à une couche de fibrinogène disposée sur ce dernier et d'une couche externe de thrombine.

7. Formulation de colle tissulaire à base de fibrine selon la revendication 5 ou 6, **caractérisée en ce qu'**une couche barrière est prévue entre la couche de fibrinogène et la couche externe de thrombine.

8. Formulation de colle tissulaire à base de fibrine selon au moins l'une des revendications 5 à 7, **caractérisée en ce que** la couche barrière est réalisée à partir de polyvinylpyrrolidone de faible poids moléculaire ou de solutions de dérivés de cellulose ou de solutions de glucides par séchage de ces solutions.

9. Formulation de colle tissulaire à base de fibrine selon au moins l'une des revendications 1 à 8, **caractérisée en ce que** le rapport thrombine/fibrinogène avec le facteur XIII se situe dans la plage de 1/10 à 1/1000, de préférence de 1/50 à 1/200.

10. Formulation de colle tissulaire à base de fibrine selon au moins l'une des revendications 1 à 9 , **caractérisée en ce que** les granulats de thrombine et/ou les granulats de fibrinogène et/ou les granulats mixtes sont pourvus d'une couche barrière externe.

11. Formulation de colle tissulaire à base de fibrine selon au moins l'une de revendications 1 à 10 **caractérisée en ce que** les protéines de thrombine et de fibrinogène ont été produites par recombinaison à l'aide de procédés technologiques génétiques ou biologiques.

12. Formulation de colle tissulaire à base de fibrine selon au moins l'une des revendications 1 à 11, **caractérisée en ce qu'**elle contient un sel de calcium.

13. Procédé de préparation d'une formulation de colle tissulaire à base de fibrine selon au moins l'une des revendications 1 à 12, **caractérisé en ce que** les solutions de protéine ou les suspensions de fibrinogène avec le facteur XIII et la thrombine sont pulvérisées dans une chambre à lit fluidisé et séchées à l'aide d'un gaz de fluidification, la température maximale du produit ne dépassant pas 50°C.

14. Procédé selon la revendication 13, **caractérisé en ce que,** dans une première étape, un concentré de fibrinogène avec le facteur XIII en solution aqueuse est pulvérisé, séché et isolé dans la chambre à lit fluidisé, et **en ce que**, dans une deuxième étape, le concentré de thrombine en solution aqueuse est pulvérisé, séché et isolé dans la chambre à lit fluidisé, et **en ce qu'**ensuite, les deux granulats obtenus sont mélangés.

15. Procédé de préparation d'une formulation de colle tissulaire à base de fibrine selon la revendication 13, **caractérisé en ce que**, dans une première étape, un concentré de fibrinogène en solution aqueuse est pulvérisé et séché dans la chambre à lit fluidisé, et **en ce qu'**ensuite, de la thrombine d'une suspension organique est pulvérisée sur ce granulat séché.

16. Procédé de préparation d'une formulation de colle tissulaire à base de fibrine selon au moins l'une des revendications 13 à 15, **caractérisé en ce que** les solutions de protéines sont pulvérisées sur un matériau support prédéterminé.
